# EUROPEAN PATENT APPLICATION

(11) **EP 4 597 117 A1**
(43) Date of publication of application: **06.08.2025**
(21) Application number: 22960995.3
(22) Date of filing: 30.09.2022
(51) Int. Cl.: G01N 35/08, G01N 37/00

(54) **FLUID PROCESSING CARTRIDGE, AND METHOD FOR RECOVERING AND AMPLIFYING NUCLEIC ACID**

(71) Applicant: Kabushiki Kaisha Mirai Genomics, Yokohama-shi, Kanagawa 230-0051 (JP)
(72) Inventor: OVECHKIN, Nikita, Yokohama-shi, Kanagawa 230-0051 (JP); PUZANKOV, Dmitrii, Yokohama-shi, Kanagawa 230-0051 (JP)
(74) Representative: f & e patent
(86) International application number: PCT/JP2022/036721
(87) International publication number: WO 2024/069936

(57) **Abstract**

The present invention relates to a fluid processing cartridge having a plurality of liquid storage tanks, a flow channel switching means, and a flow channel between each of the liquid storage tanks and the flow channel switching means. The flow channel switching means includes a rotor, and a cylinder in which the rotor is slidable while the outer peripheral surface of the rotor and the inner peripheral surface of the cylinder maintain close contact with each other, and the rotor has at least one groove on the outer peripheral surface, the at least one groove allowing a plurality of flow ports of the cylinder to be interconnected by a rotation or parallel movement of the rotor, and/or the rotor has at least one through-hole to allow the plurality of flow ports of the cylinder to be interconnected by the rotation or parallel movement of the rotor. The present invention relates to a method for recovering and amplifying nucleic acid with the cartridge. The present invention provides a cartridge and a nucleic acid recovery and amplification method that allow the amplification and detection of nucleic acid recovered from a specimen to be performed in the same device with a simple transfer means of liquid, making the device more compact.

## Description

### TECHNICAL FIELD

The present invention relates to a fluid processing cartridge and a method for recovering and amplifying nucleic acid. More particularly, the present invention relates to a fluid processing cartridge suitable for recovering, amplifying, and detecting nucleic acid from a specimen, and a method for recovering and amplifying nucleic acid with the cartridge.

### BACKGROUND ART

With the spread of COVID-19, there is a growing demand for technologies to recover, amplify and detect nucleic acid in viruses to conveniently and reliably determine the presence or absence of viral infection. In addition, even after COVID-19 problem has been resolved, human beings continue to face diseases caused by various viruses and bacteria, and therefore demands for similar technologies still remain.

The technology for amplifying and detecting nucleic acid has been almost established as a methodology thanks to the advent of PCR method and the subsequent development of various methods other than PCR method, and has become convenient enough to be used not only in laboratories but also in examination rooms in hospitals. Even so, in the case of using existing devices for amplification and detection, the amplification and detection of nucleic acid were often performed after performing manual operations to recover nucleic acid from biological samples such as viruses. However, with the spread of COVID-19, it became clear that a serious situation cannot be dealt with by using existing methods and devices for specimens brought to public health centers or examination companies. At present, it is not possible to reliably determine the presence or absence of viral infection by processing a large amount of specimens quickly and simply, regardless of location.

Patent Literature 1 discloses a portable small-sized device for amplification and detection that can more easily amplify and detect nucleic acid.
Patent Literature 1: International Publication WO 2018/123837
Patent Literature 2: International Publication WO 2002/023776

The entire description of Patent Literatures 1 and 2 is incorporated herein by reference in particular.

### SUMMARY OF INVENTION

### TECHNICAL PROBLEM

The device described in Patent Literature 1 is a device that separately recovers nucleic acid from a specimen and then amplifies and detects the recovered nucleic acid. However, in the method with this device, it is necessary to separately recover the nucleic acid from the specimen, and it is not possible to detect viruses from the specimen at once.

In contrast, the present inventors planned to use a specimen as it is without pretreating, and to perform, in a single device, recovering nucleic acid from the specimen, amplifying the recovered nucleic acid, and detecting the amplified nucleic acid. This device is intended to perform the above-mentioned processes of recovering, amplifying, and detecting nucleic acid in chambers connected by micro flow channels in a sequential and automatic manner, and to use a carrier such as a filter, taking into account the high efficiency of recovering nucleic acid.

A device for recovering nucleic acid with a carrier is known (Patent Literature 2). The device has a plurality of chambers for storing a plurality of reagent solutions and a mechanism for transferring fluid between the chambers. The fluid transfer mechanism between the chambers includes a cylindrical body in which the inside is a fluid transfer chamber and a piston that moves up and down in the cylindrical body to reduce or increase the pressure inside the cylindrical body, and has a switchable flow channel connected to other chambers at the bottom of the fluid transfer chamber, and recovers nucleic acid from a specimen while moving the fluid between the fluid transfer chamber and these other chambers by use of reducing or increasing the pressure inside the cylindrical body. However, this device needs to move the liquid by the vertical movement of the piston, and has a problem that the operation mechanism becomes complicated, and does not intend to amplify and detect the recovered nucleic acid in the same device.

An object of the present invention is to provide a cartridge that can amplify and detect the recovered nucleic acid in the same device, and that has a simple transfer means of liquid and allows the device on which the cartridge is mounted to be made more compact.

### SOLUTION TO PROBLEM

The present invention is as follows.
[1] A fluid processing cartridge comprising a plurality of liquid storage tanks, a flow channel switching means, and a flow channel between each liquid storage tank and the flow channel switching means,
   the flow channel switching means includes a rotor, and a cylinder in which the rotor is slidable while the outer peripheral surface of the rotor and the inner peripheral surface of the cylinder maintain close contact with each other, and
   the rotor has at least one groove on the outer peripheral surface, the at least one groove allowing a plurality of flow ports of the cylinder to be interconnected by a rotation or parallel movement of the rotor, and/or
   the rotor has at least one through-hole allowing a plurality of flow ports of the cylinder to be interconnected by the rotation or parallel movement of the rotor.
[2] The cartridge according to [1], wherein the groove has an opening of the through-hole from the inside of the rotor.
[3] The cartridge according to [1] or [2], wherein the rotor is a solid cylinder or has a cylindrical outer wall having a cavity therein, and the through-hole is a hole passing through the solid cylinder or a hole passing through the cylindrical outer wall.
[4] The cartridge according to any one of [1] to [3], wherein the plurality of liquid storage tanks, the flow channels and the cylinder are integrated, and the rotor is insertable through an opening of the cylinder.
[5] The cartridge according to any one of [1] to [4], wherein the rotor has openings at both ends, and the rotor has an internal space from each opening and has an internal structure portion separating two or more internal spaces.
[6] The cartridge according to any one of [1] to [5], wherein the cylinder is closed at the end on the inner side of the cylinder, or the cylinder has a nozzle extending from the inner side of the cylinder to an internal space of the rotor and is closed except for at the nozzle, the nozzle has an opening at the tip, and the nozzle is communicable with a flow channel in the cylinder.
[7] The cartridge according to any one of [1] to [6], wherein the fluid processing cartridge has a carrier in the flow channel switching means or on the way of the flow channel between the liquid storage tank and the flow channel switching means.
[8] The cartridge according to [7], wherein when the rotor has openings at both ends, and the rotor has an internal space from each opening and has an internal structure portion separating two or more internal spaces,
   the carrier in the flow channel switching means is included in the internal structure portion inside the rotor.
[9] The cartridge according to any one of [5] to [8], wherein when the rotor has openings at both ends, and the rotor has an internal space from each opening and has an internal structure portion separating two or more internal spaces,
   the internal structure portion includes a liquid receiving portion on the outer side of the cylinder, and the liquid receiving portion has a discharge channel that is communicable with the outside of the rotor.
[10] The cartridge according to [9], wherein the liquid receiving portion is funnel-shaped.
[11] The cartridge according to any one of [1] to [10], wherein the fluid processing cartridge further comprises a waste solution recovery tank connected to the flow channel switching means via a flow channel, and the waste solution recovery tank has an exhaust port.
[12] The cartridge according to [11], wherein the exhaust port is positioned above a waste solution tank inlet in the waste solution recovery tank when the fluid processing cartridge is used.
[13] The cartridge according to [11] or [12], wherein a liquid flow and an exhaust in the liquid storage tanks and in the flow channels, when the fluid processing cartridge is used, are performed by a negative pressure generated by the exhaust from the exhaust port.
[14] The cartridge according to any one of [1] to [13], wherein the fluid processing cartridge further comprises a reaction vessel connected to the flow channel switching means via a flow channel.
[15] The cartridge according to any one of [1] to [14], wherein, at least one of the plurality of liquid storage tanks and the reaction vessel have a vent hole or a vent passage leading to the vent hole, and a communication port to the vent hole of the liquid storage tank is located in the upper part of the liquid storage tank when the fluid processing cartridge is used.
[16] The cartridge according to any one of [1] to [15], wherein the flow channel switching means is capable of closing at least the reaction vessel by the positional relationship between the rotor and the cylinder.
[17] The cartridge according to any one of [1] to [16], wherein the grooves and through-holes in the flow channel switching means have structures that are capable of switching all the flow channels to an unconnected state via the flow channel switching means, by the positional relationship between the rotor and the cylinder.
[18] The cartridge according to any one of [1] to [17], wherein the rotor has one or more grooves and one or more through-holes.
[19] The cartridge according to any one of [1] to [18], wherein the plurality of liquid storage tanks is one or more specimen suspension tanks, one or more detergent tanks, one or more eluent tanks, or one or more mixing tanks, and the fluid processing cartridge further comprises one or more waste solution tanks and one or more reaction vessels.
[20] The cartridge according to any one of [1] to [19], wherein the cartridge is for use of recovering, amplifying and/or detecting nucleic acid from a specimen, and the amplification of nucleic acid is performed in the reaction vessel.
[21] A method comprising recovering and amplifying nucleic acid with the cartridge according to any one of [1] to [20].
[22] The method according to [21], wherein the rotor and the cylinder of the flow channel switching means take a position where at least the reaction vessel is closed, or a position where all the flow channels are in an unconnected state, when the amplification of nucleic acid is performed in the reaction vessel.
[23] The method according to [21] or [22], further comprising a step of detecting the amplified nucleic acid after the nucleic acid amplification operation, by optically or electrically or by surface plasmon resonance.

### ADVANTAGEOUS EFFECT OF INVENTION

According to the present invention, it is possible to provide a cartridge that can amplify and detect the recovered nucleic acid in the same device, and that has a simple transfer means of liquid and allows the device on which the cartridge is mounted to be made more compact. The use of this cartridge makes it possible to more reliably detect the target nucleic acid.

### BRIEF DESCRIPTION OF DRAWINGS

[FIG. 1] FIG. 1 shows a schematic view of an example of the fluid processing cartridge of the present invention.
[FIG. 2] FIG. 2 is a schematic explanatory diagram showing a horizontal cross section of a flow channel switching means included in an example of the fluid processing cartridge of the present invention.
[FIG. 3] FIG. 3 is a schematic explanatory diagram showing a vertical cross section of a flow channel switching means included in an example of the fluid processing cartridge of the present invention.
[FIG. 4] FIG. 4 is a schematic explanatory diagram showing a vertical cross section of a cylinder and a rotor of an example of the fluid processing cartridge of the present invention.
[FIG. 5] FIG. 5 is a schematic explanatory diagram showing a horizontal cross section of a flow channel switching means included in an example of the fluid processing cartridge of the present invention.
[FIG. 6] FIG. 6 is a schematic explanatory diagram showing a horizontal cross section of a flow channel switching means included in an example of the fluid processing cartridge of the present invention.
[FIG. 7] FIG. 7 is a schematic explanatory diagram showing a horizontal cross section of a flow channel switching means included in an example of the fluid processing cartridge of the present invention.
[FIG. 8] FIG. 8 is a schematic explanatory diagram showing a vertical cross section of a cylinder and a rotor of an example of the fluid processing cartridge of the present invention.
[FIG. 9-1] FIG. 9-1 is a schematic explanatory diagram showing a vertical cross section of a cylinder and a rotor of an example of the fluid processing cartridge of the present invention.
[FIG. 9-2] FIG. 9-2 is a schematic explanatory diagram showing a vertical cross section of a cylinder and a rotor of an example of the fluid processing cartridge of the present invention.

### EMBODIMENTS OF INVENTION

### <Fluid Processing Cartridge>

The present invention relates to a fluid processing cartridge, and the cartridge has a plurality of liquid storage tanks, a flow channel switching means, and a flow channel between each of the liquid storage tanks and the flow channel switching means. The flow channel switching means includes a rotor, and a cylinder in which the rotor is slidable while the outer peripheral surface of the rotor and the inner peripheral surface of the cylinder maintain close contact with each other. The rotor may have at least one groove on the outer peripheral surface thereof, the at least one groove allowing a plurality of flow ports of the cylinder to be interconnected by a rotation or parallel movement of the rotor. The rotor may also have at least one through-hole allowing a plurality of flow ports of the cylinder to be interconnected by the rotation or parallel movement of the rotor.

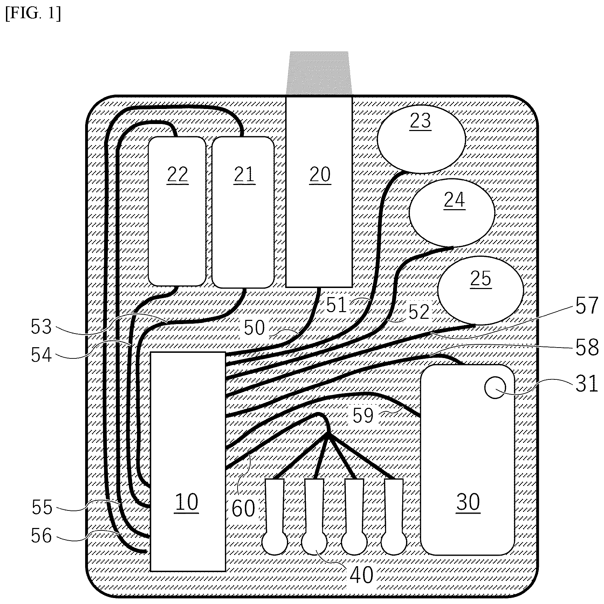

The fluid processing cartridge of the present invention will be described below with reference to FIG. 1. FIG. 1 is a schematic diagram of an example of the fluid processing cartridge of the present invention.

In FIG. 1, reference numeral 10 denotes a flow channel switching means, reference numerals 20 to 25 denote liquid storage tanks, and reference numerals 50 to 57 denote flow channels between the respective liquid storage tanks and the flow channel switching means. Reference numerals 58 and 59 denote flow channels between a waste solution recovery tank and the flow channel switching means, and reference numeral 60 denotes a flow channel between reaction vessels and the flow channel switching means. FIG. 1 shows six liquid storage tanks of 20 to 25 as an example, but the number of liquid storage tanks is not particularly limited as long as it is two or more, and may be 2, 3, 4, 5, 6, 7, 8, 9, 10 or more. Although FIG. 1 shows flow channels of 50 to 60, the number of flow channels can be appropriately selected according to the number of the liquid storage tanks, waste solution recovery tank and reaction vessel. The plurality of liquid storage tanks, the flow channel, and the cylinder in the cartridge of the present invention can be integrated, and the rotor is insertable from an opening side of the cylinder. For example, the opening of the cylinder is placed at the bottom of the cartridge (the surface on the lower side in the longitudinal direction of the flow channel switching means 10). The configuration of the rotor and the cylinder will be described later.

The flow channel switching means 10 includes the rotor, and the cylinder in which the rotor is slidable while the outer peripheral surface of the rotor and the inner peripheral surface of the cylinder maintain close contact with each other. The "slidable" for the rotor and the cylinder means that the rotor is rotatable in the cylinder and/or that the rotor is parallel-movable in the cylinder. The flow channel switching means consisting of the rotor and the cylinder is a longitudinally elongated cylindrical shape, flow channels are connected to the side peripheral surface thereof, in FIG. 1, both ends are placed so as to be up and down, but both ends can also be placed at a horizontal position or an oblique position with various angles. In order to keep the outer peripheral surface of the rotor and the inner peripheral surface of the cylinder in close contact with each other, it is preferable that the outer diameter of the rotor and the inner diameter of the cylinder are substantially equal, and the surface of the outer peripheral surface of the rotor and the surface of the inner peripheral surface of the cylinder are smooth enough to maintain the close contact. In addition, various lubricating oils may be used between the cylinder and the rotor allowing them to be in close contact, and also allowing them to rotate and be inserted and removed.

The rotor can be a solid cylinder or be a cylinder with an outer wall and a cavity therein. The rotor can have at least one groove on the outer peripheral surface thereof, the at least one groove being capable of communicating a plurality of flow ports of the cylinder by a rotational position of the rotor. The groove may be provided in the horizontal direction or the vertical direction. Although it may be provided with an incline, it is preferable to provide it in the horizontal direction or the vertical direction in consideration of an operability of the flow channel switching means 10. The length and depth of the groove can be appropriately determined in consideration of the spacing between the plurality of flow ports of the cylinder and sizes of them.

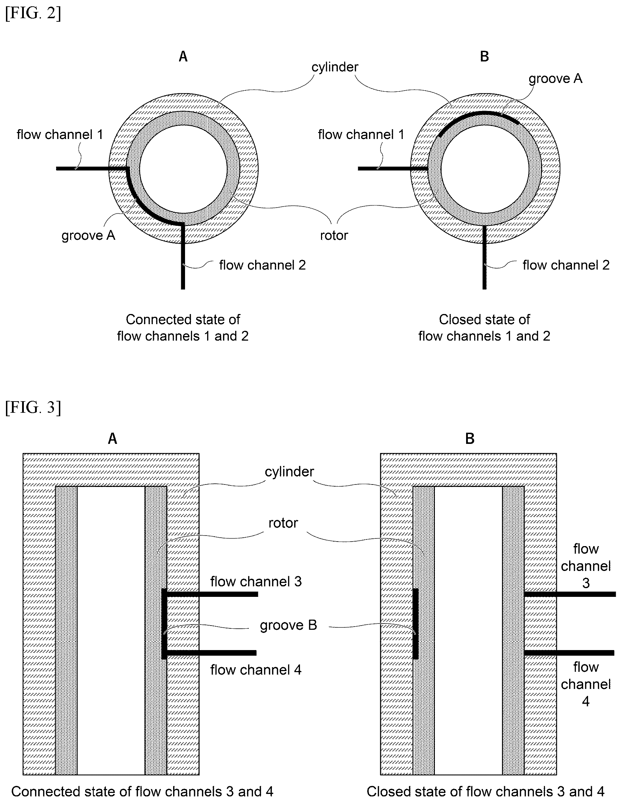

FIG. 2 is a schematic explanatory diagram showing a horizontal cross section of the rotor having a cylindrical outer wall with a cavity therein and the cylinder. An example in which the groove is provided in the horizontal direction on the outer peripheral surface of the rotor is shown. In section A, flow channels 1 and 2 are in communication with each other at both ends of groove A. Section B shows a state in which the rotor has rotated, the both ends of groove A are in a non-connected state with the openings of flow channels 1 and 2, and the communication between flow channels 1 and 2 is closed. In section A, groove A can be used to allow liquid or gas to flow between flow channels 1 and 2. In the state of section B, the communication between flow channels 1 and 2 is closed, and the flow of liquid or gas through groove A is not possible. In order to disconnect the connection between the two flow channels, the groove may be in non-contact with at least one of the two flow channels.

FIG. 3 is a schematic explanatory diagram showing a vertical cross section of the rotor and the cylinder. An example in which the groove is provided in a direction perpendicular to the outer peripheral surface of the rotor is shown. In section A, flow channels 3 and 4 are in communication with each other at both ends of groove B. Section B shows a state in which the rotor has rotated, the both ends of groove B are in a non-connected state with the openings of flow channels 3 and 4, and the communication between flow channels 3 and 4 is closed. In the state of section A, groove B can be used to allow liquid or gas to flow between flow channels 3 and 4. In the state of section B, the communication between flow channels 3 and 4 is closed, and the flow of liquid or gas through groove B is not possible.

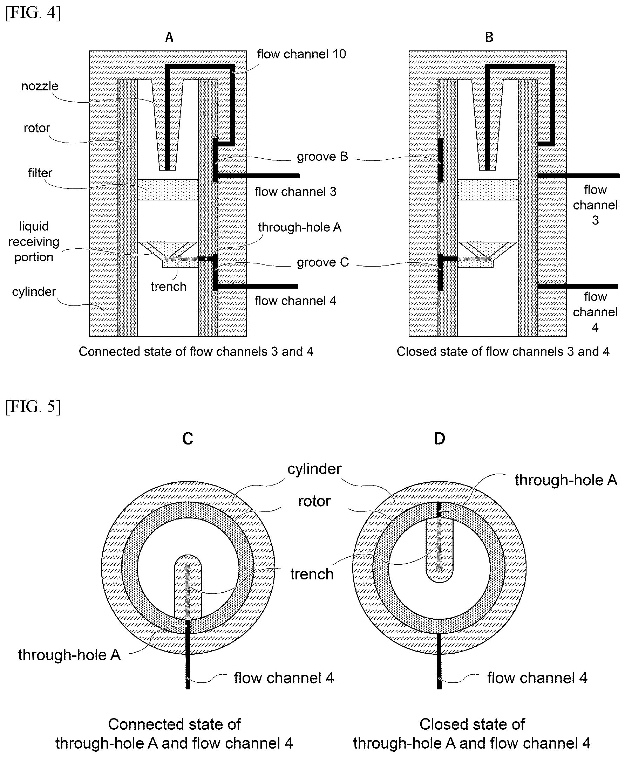

FIG. 4 is a schematic explanatory diagram showing a vertical cross section of the rotor and the cylinder. The rotor shown in FIG. 4 is in a cylindrical form, having a cylindrical outer wall with a cavity therein and having openings at both ends. The rotor within the cylinder has openings at the ends on the inner and outer sides of the cylinder and the rotor has an internal space from each opening. The internal space of the rotor can be a mere space as shown in FIG. 3 or it can have an internal structure portion that separates two internal spaces as shown in FIG. 4. The cylinder can be closed at the end on its inner side, as shown in FIG. 3, or it can have a nozzle extending from the inner side of the cylinder to the internal space in the rotor, as shown in FIG. 4, and the cylinder is closed except for at the nozzle (and optionally at the opening for inserting the rotor). The nozzle has an opening at the tip, and the nozzle is in communication with flow channel 10 in the cylinder. In section A of FIG. 4, flow channel 3 is in communication with the nozzle via groove B and flow channel 10 in the cylinder. Section B is a diagram in which the rotor has rotated to another position, and shows a state in which the connection between groove B and flow channel 3 and flow channel 10 in the cylinder has been cut off.

The fluid processing cartridge of the present invention may have a carrier in the flow channel switching means or on the way of the flow channel between the liquid storage tank and the flow channel switching means. The carrier in the flow channel switching cartridge may constitute the internal structure portion inside the rotor, and the tip of the nozzle extending from the cylinder may be located near an opposite surface of the carrier. The carrier may be any one capable of trapping nucleic acid in a specimen and eluting the trapped nucleic acid. The carrier can be, for example, a glass filter, an Affinity resin such as a normal phase column and a reversed phase column, and a gel-filtration resin, and the like. However, there is no intention to limit it to these.

The internal structure portion within the rotor may include a liquid receiving portion on the outer side of the cylinder. A space may be provided between the carrier and the liquid receiving portion, which constitute the internal structure portion. The liquid receiving portion may have a trench that is a discharge channel communicable from the liquid receiving portion to the outside of the rotor. The liquid receiving portion may be funnel-shaped. In section A of FIG. 4, the liquid receiving portion is connected to the trench and groove C, and groove C is connected to flow channel 4. Section B is a diagram in which the rotor has rotated to another position, and shows a state in which the connection between groove C and flow channel 4 has been cut off.

In the state of section A in FIG. 4, when the cartridge is used, the cartridge is installed so that the nozzle is positioned on the upper side along the gravitational axis (the vertical direction in the paper plane of FIG. 4), and the fluid processing is performed. The liquid supplied from flow channel 3 flows out from the nozzle tip, and the liquid flowing out falls onto the carrier, passes through the carrier, reaches the liquid receiving portion, and flows out of the liquid receiving portion into flow channel 4 via the trench and through-hole A. Although not shown, the destination of flow channel 4 is the waste solution recovery tank having a negative pressure, and the upper space within the rotor including the nozzle and the space between the liquid receiving portion and the carrier are closed spaces, and the liquid moves in the direction of the waste solution recovery tank by the negative pressure from the waste solution recovery tank.

FIG. 5 is a schematic explanatory diagram showing a horizontal cross section of an example of the flow channel switching means. Section C shows the trench downstream of the liquid receiving portion, through-hole A, and flow channel 4, which are in a communication state. Section C of FIG. 5 shows the state shown in FIG. 4, in which the trench, through-hole A, groove C in vertical, and flow channel 4 are in connection. Section D of FIG. 5 shows the state in which the rotor has rotated, the position of the opening of flow channel 4 of the cylinder and through-hole A of the rotor are mismatched, and the communication between flow channel 4 and the trench has been cut off.

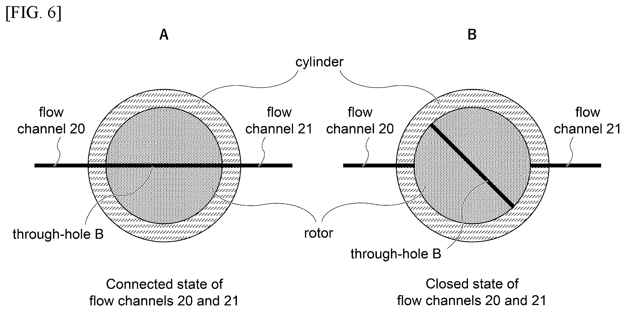

FIGS. 6 to 10 are schematic explanatory diagrams showing horizontal cross sections or vertical cross sections of examples of the flow channel switching means in which the rotor is a solid cylinder. FIG. 6 shows a through-hole B that crosses the rotor which is a solid cylinder, and flow channels 20 and 21 that can be connected to through-hole B by rotating. In section A, through-hole B is in communication with flow channels 20 and 21, and in section B, the rotor has rotated from the state of section A, and through-hole B is not in communication with flow channels 20 and 21.

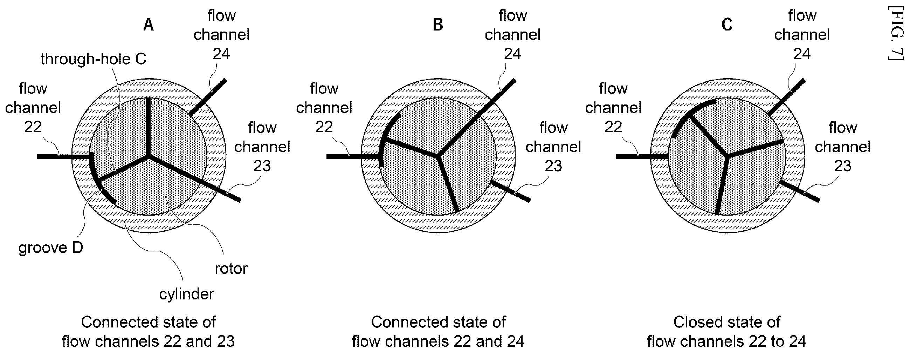

FIG. 7 shows a through-hole C that branches across the rotor which is a solid cylinder, a groove D at one end of through-hole C, and flow channels 22-24 that can be connected to through-hole C or groove D. In section A, flow channel 22 is in communication with groove D, and through-hole C is in communication with flow channel 23. In section B, the rotor has rotated from the state shown in section A, and the communication between flow channel 22 and groove D is maintained, and through-hole C is in communication with flow channel 24. In section C, the rotor has rotated from the state show in section B, and through-hole C is not in communication with any of flow channels 22-24.

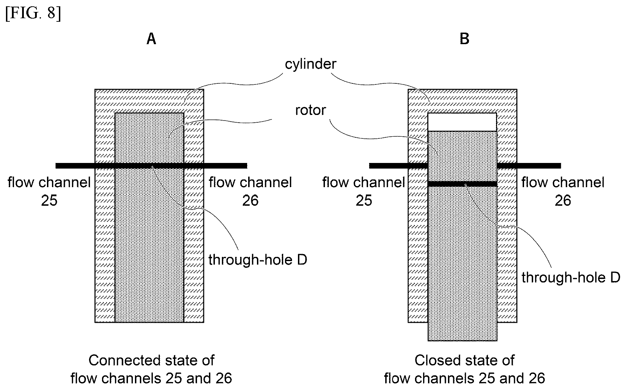

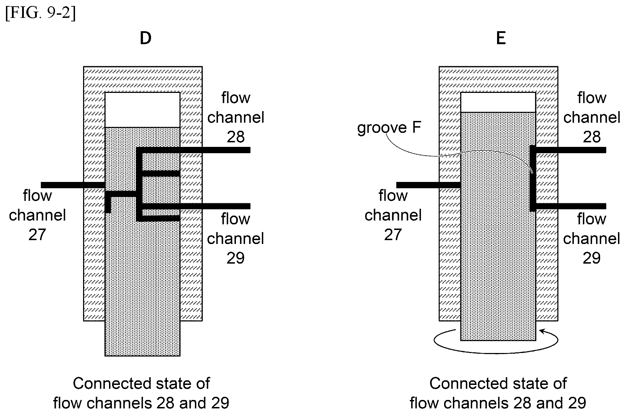

FIGS. 8 to 9-2 show examples of the parallel movement of the rotor in the cylinder. FIG. 8 shows a through-hole D that crosses the rotor which is a solid cylinder, and flow channels 25 and 26 that can be connected to through-hole D by parallel movement (linear movement) of the rotor. In section A, through-hole D is in communication with flow channels 25 and 26, and in section B, the rotor has parallel-moved from the state shown in section A, and through-hole D is not in communication with flow channels 25 and 26.

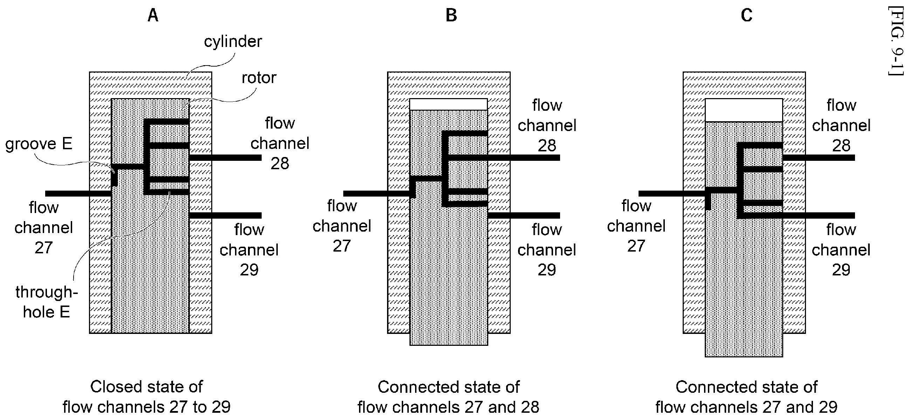

FIG. 9-1 shows a through-hole E that branches across the rotor which is a solid cylinder, a groove E at one end of through-hole E, and flow channels 27-29 that can be connected to through-hole E or groove E. In section A, through-hole E is not in communication with any of flow channels 27-29. In section B, the rotor has parallel-moved from the state shown in section A, flow channel 27 is in communication with groove E, and through-hole E is in communication with flow channel 28. In section C, the rotor has further parallel-moved from the state shown in section B, the communication between flow channel 27 and groove E is maintained, and through-hole E is in communication with flow channel 29. FIG. 9-2 is an example similar to FIG. 9-1, and in section D the rotor has further parallel-moved from the state shown in section C of FIG. 9-1, and flow channel 28 is in communication with flow channel 29 via through-hole E. Section E shows another embodiment from section D, in which the rotor has rotated, e.g., from the state shown in section C of FIG. 9-1, and flow channels 28 and 29 are interconnected by groove F.

The fluid processing cartridge of the present invention can determine flow channels to be communicated with each other by selecting the position of the groove and through-hole of the rotor and the opening of the flow channel of the cylinder as shown in FIGS. 2 to 9-2 and rotating or parallel-moving the rotor to the selected stop position, and then allows the liquid to flow between the plurality of liquid storage tanks to perform a desired processing. The rotation or parallel-movement of the rotor can be performed by operating the rotor from the outer side of the cylinder.

The fluid processing cartridge of the present invention may further comprise a waste solution recovery tank connected to the flow channel switching means via a flow channel, and may have an exhaust port in the waste solution recovery tank. In FIG. 1, the waste solution recovery tank is indicated by reference 30, and the waste solution recovery tank 30 has exhaust port 31 therein. The waste solution recovery tank 30 is connected to the flow channel switching means 10 via flow channel 59. The exhaust port 31 is connected to an exhaust cartridge provided outside the fluid processing cartridge, for example, a suction pump, and can guide liquid or gas to the waste solution recovery tank 30 by setting the waste solution recovery tank 30 to a negative pressure and further by setting the insides of liquid storage tanks and reaction vessels described later, which are connected to the fluid processing cartridge 10, to a negative pressure through the waste solution recovery tank 30 and flow channel 59. The waste solution recovery tank 30 can be set to the negative pressure with the exhaust cartridge, the liquid storage tank to be communicated with the waste solution recovery tank 30 can be selected with the flow channel switching means, and thereby it is possible to easily and conveniently perform a liquid processing with a plurality of liquid storage tanks and reaction vessel that includes transferring liquid and gas. One feature of the cartridge of the present invention is that the liquid and gas are transferred by using a negative pressure due to the exhaust from the exhaust port provided in the waste solution recovery tank 30. When the cartridge is used, the cartridge is placed such that the exhaust port is located above the waste solution tank inlet in the waste solution recovery tank. This is to prevent liquid leakage from the exhaust port.

The fluid processing cartridge of the present invention may further comprise reaction vessel 40 connected to the flow channel switching means via a flow channel. The reaction vessel is used to store the liquid containing nucleic acid processed by the cartridge of the present invention, to amplify the nucleic acid and to detect the amplified nucleic acid.

At least a portion of the plurality of liquid storage tanks and the reaction vessel may have vent holes or vent passages leading to the vent holes. In the embodiment shown in FIG. 1, any one or all of the liquid storage tanks 20 to 25 and the reaction vessels 40 have vent holes or vent passages leading to the vent holes, not shown. The liquid storage tanks having vent holes or vent passages leading to the vent holes are connected to the flow channel switching means 10 which is connected to the waste solution recovery tank 30 via flow channel 59, and upon making a negative pressure in the tanks, the liquid or gas can be easily guided to the waste solution recovery tank 30. The reaction vessels 40 having vent holes or vent passages leading to the vent holes are connected to the flow channel switching means 10 which is connected to the waste solution recovery tank 30 via flow channel 59, and upon making a negative pressure in the vessels, the liquid or gas can be easily guided to the the reaction vessels 40. Alternatively, although not shown, the reaction vessels 40 may have exhaust ports similar to the exhaust port provided in the waste solution recovery tank 30. Fluid and/or gas may be transferred to the reaction vessels 40 by exhausting from the exhaust port to make a negative pressure in the reaction vessels 40.

The fluid processing cartridge of the present invention has a number of grooves and through-holes corresponding to the number of liquid storage tanks and the presence or absence of a waste solution recovery tank and carriers.

Hereinafter, the configuration and operation of the fluid processing cartridge of the present invention will be further described with an example having a filter that is a carrier in the rotor. The cartridge of this mode comprises one specimen suspension tank, two detergent tanks, one eluent tank, one reaction solution tank, and one mixing tank as the plurality of liquid storage tanks, and further comprises one waste solution tank and one reaction vessel.

### Step 1 (Filtering step)

### Liquid: specimen suspension tank → flow channel switching means (nozzle → filter) → waste solution tank

### Gas: vent hole of specimen suspension tank → specimen suspension tank

In step 1, the rotor of the flow channel switching means is at a position where the flow channel from the specimen suspension tank communicates with the flow channel in the cylinder (corresponding to flow channel 10 shown in FIG. 4) and the nozzle via the groove of the flow channel switching means. This flow channel connection is a configuration from flow channel 3 to the nozzle shown in FIG. 4A. In addition, the flow channel connection, which takes the position where the through-hole and the flow channel communicate from below the liquid receiving portion to the waste solution tank via the groove, corresponds to a configuration shown in FIGS. 4A and 5C. The inside of the waste solution tank is in a negative pressure, and the liquid flows from the specimen suspension tank into the flow channel switching means via the flow channel, passes through the filter in the flow channel switching means, and reaches the waste solution tank in a negative pressure state via the flow channel between the flow channel switching means and the waste solution tank. If the liquid from the specimen suspension tank contains nucleic acid, the nucleic acid is trapped in the filter.

### Step 2 (Cleaning step)

### Liquid: detergent tank → flow channel switching means (nozzle → filter) → waste solution tank

### Gas: vent hole of detergent tank → detergent tank

In step 2, the rotor in the flow channel switching means is rotated to take a position where the flow channel from the detergent tank communicates with the flow channel in the cylinder (corresponding to flow channel 10 shown in FIG. 4) and the nozzle via the groove of the flow channel switching means and where the through-hole from below the liquid receiving portion communicates with the waste solution tank. Since the flow channel connected to the groove of the flow channel switching means is a flow channel from the detergent tank, the height or the position on the outer periphery of the flow channel and the groove differs from in step 1 (the flow channel connected to the groove of the flow channel switching means in step 1 is a flow channel from the specimen suspension tank), but this flow channel connection is the same as the configuration shown in FIGS. 4A and 5C. Thus, the detergent in the detergent tank flows from the detergent tank to the nozzle in the flow channel switching means via the flow channel, passes through the filter from the nozzle, and reaches the waste solution tank in the negative pressure state via the flow channel between the liquid receiving portion and the waste solution tank. The detergent from the detergent tank cleans the filter.

When two detergent tanks are used, the above-described operation is repeated using two different detergent tanks, and thereby the cleaning can be performed more thoroughly. However, although the positioning of the flow channel switching means corresponds to the configuration as shown in FIGS 4A and 5C, the height or position on the outer periphery of the flow channel and groove is different from in step 1 and in the first time of step 2.

### Step 3 (Drying step)

### Gas: vent hole of detergent tank → flow channel switching means (nozzle → filter) → waste solution tank

After one or two cleaning steps have been completed, the flow channel switching means is positioned the same as in step 2, and the outside air is taken in from the vent hole of the detergent tank, and the outside air passes through the filter in the flow channel switching means and reaches the waste solution tank in the negative pressure state. Thus, the filter is dried.

### Step 4 (Elution step)

### Liquid: eluent tank → flow channel switching means (nozzle → filter) → mixing tank

### Gas: mixing tank → flow channel switching means → waste solution tank

In step 4, the rotor of the flow channel switching means rotates to take the position where the flow channel from the eluent tank communicates with the flow channel in the cylinder (corresponding to flow channel 10 as shown in FIG. 4) and the nozzle via the groove of the flow channel switching means and where the opening from below the liquid receiving portion communicates with the through-hole communicating with the mixing tank. This flow channel connection differs from steps 1 to 3 in the height or the position on the outer periphery of the flow channel and the groove, but it is the embodiment shown in FIGS. 4A and 5C. Furthermore, in this step, the gas also flows from the mixing tank to the waste solution tank via the flow channel switching means. The flow channel from the mixing tank is in communication with the groove of the rotor, and the same groove is in communication with the flow channel communicating with the waste solution tank. This flow channel connection corresponds to the state as shown in FIG. 2A. Thus, the eluent in the eluent tank flows from the eluent tank into the nozzle via the flow channel, passes through the filter, and reaches the mixing tank. The eluent from the eluent tank elutes and cleans the nucleic acid trapped in the filter. Furthermore, the gas from the mixing tank is sucked into the waste solution tank in the negative pressure state via the flow channel switching means.

### Step 5 (Reaction solution supplying step)

### Liquid: reaction solution tank → flow channel switching means → mixing tank

### Gas: mixing tank → flow channel switching means → waste solution tank

In step 5, the rotor of the flow channel switching means rotates to take the position where the flow channel from the reaction solution tank is communicated with the groove of the flow channel switching means and where the same groove of the flow channel switching means is communicated with the flow channel communicating to the mixing tank. This flow channel connection corresponds to the state as shown in FIG. 2A. Furthermore, this positioning allows also that a flow channel from the mixing tank, which is a flow channel for gas, is communicated with the groove of the flow channel switching means, and that the same groove of the flow channel switching means is communicated with the flow channel communicating with the waste solution tank. This flow channel connection also corresponds to the state as shown in FIG. 2A. As a result, the reaction solution in the reaction solution tank reaches the mixing tank from the reaction solution tank via the flow channel and the flow channel switching means. **In** the mixing tank, the eluent, the reaction solution, and the eluted nucleic acid are mixed. Furthermore, the gas from the mixing tank is sucked into the waste solution tank having the negative pressure via the flow channel switching means.

### Step 6 (Reaction vessel charging step)

### Liquid: mixing tank → flow channel switching means → reaction vessel

### Gas: reaction vessel → flow channel switching means → waste solution tank

In step 6, the rotor of the flow channel switching means rotates to take the position where the flow channel from the mixing tank is communicated with the groove of the flow channel switching means and where the same groove of the flow channel switching means is communicated with the flow channel communicating with the reaction vessel. This flow channel connection corresponds to the state as shown in FIG. 2A. Furthermore, this positioning allows also that the flow channel from the exhaust port of the reaction vessel, which is a flow channel for gas, is communicated with the groove of the flow channel switching means, and that the same groove of the flow channel switching means is communicated with the flow channel communicating with the waste solution tank. This flow channel connection corresponds to the state as shown in FIG. 3A. As a result, the reaction solution in the mixing tank reaches the reaction vessel from the mixing tank through the flow channel via the groove of the flow channel switching means. The mixed reaction solution from the mixing tank is transferred to the reaction vessel. Furthermore, the gas from the reaction vessel is sucked into the waste solution tank in the negative pressure state via the flow channel switching means.

The mixed reaction solution transferred to the reaction vessel is then subjected to the step of amplifying the nucleic acid in the solution.

The flow channel switching means can have a structure of the groove and the through-hole, the structure being capable of taking a positional relationship between the rotor and the cylinder where at least the reaction vessel is closed or all the flow channels become an unconnected state via the flow channel switching means. After the mixed reaction solution is transferred to the reaction vessel, at least the reaction vessel can be closed, or all the flow channels can become the unconnected state via the flow channel switching means. Subsequently, the nucleic acid in the solution is subjected to the step of amplification. By closing at least the reaction vessel or disconnecting all the flow channels, the nucleic acid amplification and subsequent detection can be performed avoiding contamination in the reaction vessel.

The cartridge of the present invention can be used to recover, amplify and/or detect nucleic acid from a specimen.

### <Method for Amplifying Nucleic Acid >

The present invention encompasses a method for amplifying the target nucleic acid of a specimen with the fluid processing cartridge of the present invention as described above, and this method can also comprise detecting the amplified nucleic acid during the nucleic acid amplification.

The nucleic acid to be amplified is not particularly limited but may be, for example, RNA or DNA. The nucleic acid amplification reaction can be an isothermal amplification reaction or a thermocycle amplification reaction of nucleic acid. The isothermal amplification reaction is, for example, a LAMP method or a SmartAmp method. The thermocycle amplification reaction can be a PCR amplification reaction.

The enzyme for nucleic acid amplification is not particularly limited but may be, for example, an enzyme for the isothermal amplification reaction of nucleic acid or an enzyme for the thermocycle amplification reaction. The isothermal amplification reaction of nucleic acid can be performed, for example, the LAMP method or SmartAmp method for nucleic acid, and can be a nucleic acid amplification reaction with enzyme utilizing a strand displacement reaction.

A known polymerase, which is an enzyme for nucleic acid amplification reactions having a strand displacement activity, can be used as the enzyme. Examples of polymerase include that described in International Publication WO 2004/040019, but there is no intend to be limited thereto. The polymerase having the strand displacement activity can also be DNA polymerase (Aac), which is disclosed in International Publication WO 2009/054510 (JP patent 4450867).

Any polymerase that is used for the nucleic acid amplification reaction having the strand displacement activity can be used appropriately, whether it is normal temperature type, mesophilic, or thermostable. In addition, the polymerase can be either its natural form or a mutant form that is artificially mutated. Examples of such an polymerase include DNA polymerase. Examples of such DNA polymerase include mutants lacking 5'→3' exonuclease activity of DNA polymerases derived from thermophilic Bacillus bacteria such as Bacillus stearothermophilus (hereinafter referred to as "B. st") and Bacillus caldotenax (hereinafter referred to as "B. ca"), and the Klenow fragment of DNA polymerase I derived from escherichia coli (E. coli), and the like. Examples of DNA polymerase used in nucleic acid amplification reactions further include Vent DNA polymerase, Vent (Exo-) DNA polymerase, Deep Vent DNA polymerase, Deep Vent (Exo-) DNA polymerase, Φ29 phage DNA polymerase, MS-2 phage DNA polymerase, Z-Taq DNA polymerase, Pfu DNA polymerase, Pfu turbo DNA polymerase, KOD DNA polymerase, 9°Nm DNA polymerase, Therminator DNA polymerase, Taq DNA polymerase, and the like.

If the nucleic acid to be amplified is RNA, a reverse transcriptase can be used in addition to DNA polymerase, or a DNA polymerase having a reverse transcriptase activity can be used as DNA polymerase. The reverse transcriptase is not particularly limited as long as it has cDNA synthetic activity using RNA as a template, and examples thereof include reverse transcriptases of various origins, such as avian myeloblastosis virus-derived reverse transcriptase (AMVRTase), Rous-associated virus 2 reverse transcriptase (RAV-2RTase), and Moloney murine leukemia virus-derived reverse transcriptase (MMLV RTase). Examples of DNA polymerase having also the reverse transcriptase activity include BcaBEST DNA polymerase, Bca (exo-) DNA polymerase, Tth DNA polymerase, and the like.

The primer is appropriately selected according to the enzyme for nucleic acid amplification reaction. When the enzyme for nucleic acid amplification reaction is for a nucleic acid amplification reaction using the strand displacement reaction, examples of the primer include primers described in International Publication WO2004/040019, Japan Patent Publication JP2009-171935, Japan Patent Publication JP2011-50380 and the like.

The method of the present invention can further comprise a step of detecting the amplified nucleic acid after the nucleic acid amplification operation, by optically or electrically or by surface plasmon resonance. When a fluorogenic primer is used as the primer, the detection of the amplified nucleic acid can be performed by utilizing the label of the fluorogenic primer. When an exciton primer or an exciton probe is used in the amplification reaction, the detection of the amplified nucleic acid can be performed by utilizing the exciton effect. The method for optically detecting nucleic acid may be also a method utilizing an intercalating dye.

In the method of the present invention, it is preferable to perform the nucleic acid amplification reaction by the SmartAmp method or LAMP method and to detect the label with the exciton primer or exciton probe. Alternatively, it is preferable to perform the nucleic acid amplification reaction by the PCR method and to detect the label with the exciton primer or exciton probe.

Following the nucleic acid amplification reaction, a nucleic acid melting curve can be drawn, and the melting curve can be used to determine properties of the amplified product, such as whether it is a false positive or a true positive.

### INDUSTRIAL APPLICABILITY

The present invention is useful in fields related to a recovery, an amplification, and a detection of nucleic acid from a specimen.

### REFERENCE SIGNS LIST

- 10: Flow channel switching means
- 20 to 25: Liquid storage tanks
- 30: Waste solution recovery tank
- 31: Exhaust port
- 40: Reaction vessel
- 50 to 60: Flow channels

## Claims

1. A fluid processing cartridge comprising a plurality of liquid storage tanks, a flow channel switching means, and a flow channel between each liquid storage tank and the flow channel switching means,
the flow channel switching means includes a rotor, and a cylinder in which the rotor is slidable while the outer peripheral surface of the rotor and the inner peripheral surface of the cylinder maintain close contact with each other, and
the rotor has at least one groove on the outer peripheral surface, the at least one groove allowing a plurality of flow ports of the cylinder to be interconnected by a rotation or parallel movement of the rotor, and/or
the rotor has at least one through-hole allowing a plurality of flow ports of the cylinder to be interconnected by the rotation or parallel movement of the rotor.

2. The cartridge according to claim 1, wherein the groove has an opening of the through-hole from the inside of the rotor.

3. The cartridge according to claim 1 or 2, wherein the rotor is a solid cylinder or has a cylindrical outer wall having a cavity therein, and the through-hole is a hole passing through the solid cylinder or a hole passing through the cylindrical outer wall.

4. The cartridge according to any one of claims 1 to 3, wherein the plurality of liquid storage tanks, the flow channels and the cylinder are integrated, and the rotor is insertable through an opening of the cylinder.

5. The cartridge according to any one of claims 1 to 4, wherein the rotor has openings at both ends, and the rotor has an internal space from each opening and has an internal structure portion separating two or more internal spaces.

6. The cartridge according to any one of claims 1 to 5, wherein the cylinder is closed at the end on the inner side of the cylinder, or the cylinder has a nozzle extending from the inner side of the cylinder to an internal space of the rotor and is closed except for at the nozzle, the nozzle has an opening at the tip, and the nozzle is communicable with a flow channel in the cylinder.

7. The cartridge according to any one of claims 1 to 6, wherein the fluid processing cartridge has a carrier in the flow channel switching means or on the way of the flow channel between the liquid storage tank and the flow channel switching means.

8. The cartridge according to claim 7, wherein when the rotor has openings at both ends, and the rotor has an internal space from each opening and has an internal structure portion separating two or more internal spaces,
the carrier in the flow channel switching means is included in the internal structure portion inside the rotor.

9. The cartridge according to any one of claims 5 to 8, wherein when the rotor has openings at both ends, and the rotor has an internal space from each opening and has an internal structure portion separating two or more internal spaces,
the internal structure portion includes a liquid receiving portion on the outer side of the cylinder, and the liquid receiving portion has a discharge channel that is communicable with the outside of the rotor.

10. The cartridge according to claim 9, wherein the liquid receiving portion is funnel-shaped.

11. The cartridge according to any one of claims 1 to 10, wherein the fluid processing cartridge further comprises a waste solution recovery tank connected to the flow channel switching means via a flow channel, and the waste solution recovery tank has an exhaust port.

12. The cartridge according to claim 11, wherein the exhaust port is positioned above a waste solution tank inlet in the waste solution recovery tank when the fluid processing cartridge is used.

13. The cartridge according to claim 11 or 12, wherein a liquid flow and an exhaust in the liquid storage tanks and in the flow channels, when the fluid processing cartridge is used, are performed by a negative pressure generated by the exhaust from the exhaust port.

14. The cartridge according to any one of claims 1 to 13, wherein the fluid processing cartridge further comprises a reaction vessel connected to the flow channel switching means via a flow channel.

15. The cartridge according to any one of claims 1 to 14, wherein, at least one of the plurality of liquid storage tanks and the reaction vessel have a vent hole or a vent passage leading to the vent hole, and a communication port to the vent hole of the liquid storage tank is located in the upper part of the liquid storage tank when the fluid processing cartridge is used.

16. The cartridge according to any one of claims 1 to 15, wherein the flow channel switching means is capable of closing at least the reaction vessel by the positional relationship between the rotor and the cylinder.

17. The cartridge according to any one of claims 1 to 16, wherein the grooves and through-holes in the flow channel switching means have structures that are capable of switching all the flow channels to an unconnected state via the flow channel switching means, depending on the positional relationship between the rotor and the cylinder.

18. The cartridge according to any one of claims 1 to 17, wherein the rotor has one or more grooves and one or more through-holes.

19. The cartridge according to any one of claims 1 to 18, wherein the plurality of liquid storage tanks is one or more specimen suspension tanks, one or more detergent tanks, one or more eluent tanks, or one or more mixing tanks, and the fluid processing cartridge further comprises one or more waste solution tanks and one or more reaction vessels.

20. The cartridge according to any one of claims 1 to 19, wherein the cartridge is for use of recovering, amplifying and/or detecting nucleic acid from a specimen, and the amplification of nucleic acid is performed in the reaction vessel.

21. A method comprising recovering and amplifying nucleic acid with the cartridge according to any one of claims 1 to 20.

22. The method according to claim 21, wherein the rotor and the cylinder of the flow channel switching means take a position where at least the reaction vessel is closed, or a position where all the flow channels are in an unconnected state, when the amplification of nucleic acid is performed in the reaction vessel.

23. The method according to claim 21 or 22, further comprising a step of detecting the amplified nucleic acid after the nucleic acid amplification operation, by optically or electrically or by surface plasmon resonance.
